(19) Europäisches Patentamt
European Patent Office
Office européen des brevets

(11) EP 2 123 606 A1

(12) EUROPEAN PATENT APPLICATION

(43) Date of publication:
25.11.2009 Bulletin 2009/48

(51) Int Cl.:
$C01G\ 23/047^{(2006.01)}$    $A61K\ 8/18^{(2006.01)}$

(21) Application number: 08156453.6

(22) Date of filing: 19.05.2008

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(71) Applicant: KEMIRA PIGMENTS OY
00180 Helsinki (FI)

(72) Inventors:
• Latva-Nirva, Esa
40489 Düsseldorf (DE)

• Dahms, Gerd
47138 Duisburg (DE)
• Jung, Andreas
47198 Duisburg (DE)
• Fiebrig, Benjamin
46145 Oberhausen (DE)

(74) Representative: Svensson, Johan Henrik
Berggren Oy Ab
P.O. Box 16
00101 Helsinki (FI)

(54) **Ultrafine titanium dioxide nanoparticles and dispersions thereof**

(57)    The invention relates to Membrane Structured Solid Nanoparticles (MSSNs) and dispersions thereof and uses thereof. These MSSNs have a combination of active compound vehicles and emulsifiers as such to form membranes which infiltrate the entire nanoparticles so that there are emulsifiers in the interior and on the surface of said nanoparticles. The active compound in these structures comprises ultrafine titanium dioxide which is well dispersed and evenly devided therein.

**Figure 1**. UV/VIS absorption behaviour of the ultrafine $TiO_2$ active compound used in the present invention.

**Description**

The invention relates to membrane structured solid ultrafine titanium oxide containing nanoparticles and to dispersions comprising these nanoparticles. Furthermore, the invention relates to the preparation methods thereof and uses thereof.

**Background**

Pigmentary $TiO_2$, or $TiO_2$ as a white pigment is well known for its use in decorative cosmetic, paints, etc. It produces a non-transparent white film due to the pigment size with a crystal size of about typically 200 nm, more generally in the range of 180 nm to 250 nm. Due to high opacity and low UV-shielding properties pigmentary $TiO_2$ can not be used as physical UV filters in for example sunscreen products, skin care and make-up formulations. For these applications besides of UV-shielding one of the most important features is the transparency.

A totally different type of $TiO_2$ is ultrafine $TiO_2$ which is also known as microcrystalline, microfine, nanosized, micronized or attenuation grade $TiO_2$, referring to the most essential physical feature thereof, namely the small crystal size. Ultrafine $TiO_2$ which was invented not so long ago, at the late 80's has approximately one-tenth the crystal size of pigmentary $TiO_2$.

Ultrafine $TiO_2$ combines the high visual transparency with high shielding against UV light which distinguishes it clearly from the properties of pigmentary $TiO_2$. As a shielding material, ultrafine titanium oxide has a further advantage of being a highly safe material in chemical sense. The main difference between ultrafine and pigmentary $TiO_2$ is the crystal size. The cystal size of ultrafine $TiO_2$ is around 20 nm. Pigmentary $TiO_2$ scatter visible light most efficiently imparting good hiding power, whereas ultrafine $TiO_2$ scatter and reflect UV light efficiently and transmit visible wavelengths through the cystal explaining its transparency.

Several patent documents have been disclosed on the preparation of ultrafine $TiO_2$ form by vapour and liquid phase methods dealing with various aspects of the processability and cystal properties such as crystal form, cystal size, particle size and size distribution and dispersability. For example EP444798 describes a preparation method for producing efficiently rutile crystal form with crystal size less than 100 nm having a narrow size distribution enabling an easy-to-handle product. Because the band gap of rutile is lower than that of anatase, rutile has been considered to be preferable for ultraviolet light-shielding use.

In the manufacturing of titanium oxide, the titanium oxide must be heavily cracked or pulverized for obtaining ultrafine particulates. Moreover, in titanium oxide having high rutile content, the ultrafine particulates do not have sufficient specific surface area and are insufficient in dispersibility, a quality which is desired in various uses such as cosmetics. The major problem of dispersibility restricts or complicates the use and handling of ultrafine $TiO_2$ and originates from the agglomeration tendency of the material. These difficulties result in degradation of the desired final product properties such as stability, in material inefficiency, diminished transparency, diminished UV-shielding and poor film forming uniformity in the composition wherein the ultrafine $TiO_2$ is used. Ultrafine titanium dioxide is described in the article of H. Aro and G. Dahms in Compatibility of micro-fine titanium dioxide with organic UV filters, Cosmetic and Toiletries Manufacture Worldwide, 2004, p. 115-118 to provide a triple action - absorption, scattering and reflecting of UV light - in for example sunscreen products. As soon as UV light hits the titanium dioxide crystal the UV light is partly absorbed, scattered and reflected. Thus the efficiency of ultrafine titanium dioxide not only depends on the crystal size, but on the number of nonaggregated particles, as well. With an increasing number of aggregated particles the efficacy of titanium dioxide decreases. This indicates that the dispersion degree of titanium dioxide in sunscreen emulsions is also the key factor in its efficacy.

Active pharmaceutical, cosmetic and/or food technology compounds are frequently encapsulated in active compound vehicles in order to obtain targeted release of the active compound or to protect it against chemical decomposition. The active compound vehicle may be adapted to the particular application and allows appropriate metering and release of the active compound.

In the past, solid lipid nanoparticles, denoted SLN, were developed. This technology invoves hot-melt with high pressure homogenization or high speed grinding and cold homogenization. For example, one specific method for producing SLN dispersions is described in US 4 880 634, wherein the lipid nanopellets are produced by dispersing the melted lipid with water, using a high-speed agitator. The desired particle size distribution is subsequently set by means of an ultrasound treatment. Stirring takes place generally at speeds in the region of 20000 $min^{-1}$. A review of the use of solid lipid nanoparticles as carriers for active pharmaceutical and cosmetic compounds can be found in J. Microencapsulation, 1999, vol. 16, No. 6, pages 751 to 767.

The production of solid lipid nanoparticles with a small average particle diameter using SLN was found costly and inconvenient, since it requires the use of high-pressure homogenizers. Simple stirring at high speed produces only relatively large average particle diameters of 3 $\mu$m. The load capacity of the solid lipid particles is limited, and their morphology cannot always be readily controlled. Specific surface modifications are difficult to bring about.

WO2004082666 discloses a system of solid membrane structured nanoparticles (MSSNs) which in comparison to e.g. SLN nanoparticles have a higher loading capacity, allow a greater selection of active compound vehicles and surfactants, and can be present at high concentrations in dispersions. This method for producing a solid nanoparticle dispersion avoids the drawbacks of the earlier methods and is neither costly nor inconvenient to implement. In particular, small particle diameters should be obtained for a low mechanical mixing effort. It provides solid nanoparticle dispersions which have a high loading capacity, permit a wide range of active compound vehicles and emulsifiers, and allow surface modifications. An aqueous vehicle dispersion is thus provided comprising in particular MSSNs, wherein there are solid active compound vehicle particles which are based on wax, polymer or lipid, having an average diameter in the range from 10 to 10000 nm, and comprising at least one active pharmaceutical, cosmetic and/or food technology compound, fragrance or flavour. In more detail, a method for producing the aqueous vehicle dispersion is claimed comprising

a) mixing the active compound with the wax-, polymer- or lipid-based active compound vehicle and at least one emulsifier which leads in stage b) to the formation of a lyotropic liquid-crystalline mixed phase, at a temperature above the melting or softening point of the active compound vehicle, to form a phase B

b) mechanically mixing the phase B with an aqueous phase A, which may comprise an emulsifier, at a temperature above the melting or softening point of the active compound vehicle, the weight ratio of phase B to phase A being 1:5 to 5:1, without high-pressure homogenization, to form a--preferably gellike--lyotropic liquid-crystalline mixed phase,

c) diluting the mixed phase with an aqueous phase, which may comprise an emulsifier, at an aqueous-phase temperature which is below the melting or softening point of the active compound vehicle, for example at least 5°C below, preferably at least 15°C below, with stirring and without high-pressure homogenization, to a desired final concentration of the dispersion.

Furthermore, solid MSSNs of an average diameter of 10 to 10000 nm producible by the described method are claimed as well as uses of such dispersions in drug, cosmetic or food additives.

In contradiction to SLN, emulsifiers in MSSNs are present in the interior of the particles. The entire particles are composed of a membrane or membranes, whereas in SLN a solid core of the active compound vehicle is surrounded by a layer of emulsifier. Consequently, the nano-particles essentially independently of the scale in which they are viewed, have a uniform construction comprising membrane structures. In essence, MSSNs compared to SLNs are distinguished by a membrane structuring indispersed throughout the particles. Consequently there is a substantially larger membrane surface area into which active compounds can be embedded. It is therefore possible to introduce large amounts i.e. up to 70% by weight of active pharmaceutical, cosmetic and/or food technology compounds into the membranes or into the nanoparticles. These active compounds are stored not only in the surface region of the nanoparticles in the membranes, but throughout the particles. This enables active compounds to be released in a highly targeted way, including release over a prolonged period of time. The nanoparticles or lipid particles therefore constitute a membrane which infiltrates the entire particles. This mutual infiltration is characteristic for MSSNs.

WO2004082666 further discloses that it is also possible to incorporate pigmentary inorganic solids such as $TiO_2$ and ZnO into the active compound vehicles. Such products would be ideal for use in drug industry, cosmetic industry or in food additives. In these applications the role of inert, pigmentary $TiO_2$ is to bring about a desired whiteness for the products. For example, in decorative cosmetics the use of pigmentary $TiO_2$ is well known for its effect of producing a non-transparent white film on the skin.

A lot of energy is required to achieve small enough particle sizes with conventional methods. In formulating dispersions by for example SLN there is a need to use high speed homogenizers, such as high shear dispersing at 26000 rpm and homogenization at 300 bar as disclosed in J.R. Villalobos and C.C. Mueller-Goymann in Proc. Int. Meeting on Pharm. Biopharm., and Pharm. Tech., Nuremberg 15/18, March 2004. Homogenization may further be assisted by the aid of high processing temperatures and/or pressures wherein the required energy input is high and equipment demanding.

Formulations using ultrafine $TiO_2$ are much more demanding than formulating with chemical sunscreens. To make ultrafine $TiO_2$ fully compatible with a liquid heterogenous O/W, W/O, OWO, etc. system surface phenomena and colloid characteristics require careful optimization for each specific application. It is very typical that emulsions will break down i.e. flocculation or coagulation of the ultrafine $TiO_2$ will occur due to incompatibility of the selected media. This incombatibility may become apparent in several ways and by different intensities, for example, as a change in colour, precipitation and phase segregation. This segregation may be total phase segregation or various degrees of settling or agglomeration. Or, if the dispersion is stable as freshly prepared it might not withstand longer storage periods and have a short shelf life in the final formulations. As emulsions are thermodynamically unstable and maintain their structure by kinetic hindrance the role of emulsifiers to be applied and emulgation techniques to be used are the most important factors to obtain as small droplet sizes as possible.

The object of the present invention is to provide a solution to the above described problems related to the use of ultrafine titanium dioxide by providing stable ultrafine $TiO_2$ containing dispersions.

An other object of the present invention is to provide ultrafine $TiO_2$ containing stable dispersions wherein $TiO_2$ undergoes considerably less agglomeration and which are highly dispersible in selected formulations.

Yet, a further object of the present invention is to provide a method for the manufacture of these dispersions.

**Short description of the invention**

The present inventions provides membrane structured solid nanoparticles containing ultrafine titanium dioxide as defined by claim 1.

The present invention provides furthermore an aqueous dispersion comprising these novel membrane structured solid nanoparticles as defined by claim 7 and a method for producing them as defined by claim 12.

It was surprisingly found that ultrafine $TiO_2$ could be successfully incorporated as an active substance in fine particulate form into an MSSN structure despite of its strong tendency to agglomerate. In the present invention the ultrafine $TiO_2$ is incorporated into a gel or wax type network as a very finely and uniformly devided particulate powder leading to excellent product properties in various applications. These type of dispersions enable a wide range of applications wherein efficient $TiO_2$ concentration and good transparency are of outmost importance. The evenness of the distribution of ultrafine $TiO_2$ throughout an emulsion controls for example the SPF contribution produced by a creamy application. Products according to present invention were found to provide stable emulsions with good SPF by a low amount of $TiO_2$.

Figure 1. UV/VIS absorption behaviour of an ultrafine $TiO_2$ active compound used in the present invention.

Figure 2a. A schematic graph of the particle size distribution of a conventional dispersion containing agglomerated ultrafine $TiO_2$.

Figure 2b. A schematic graph of the particle size distribution of a dispersion containing ultrafine $TiO_2$ in a dispersion of $TiO_2$-MSSNs according to the invention.

Figure 3. A schematic figure of the structure of a $TiO_2$-MSSN according to the invention.

Figure 4. The particle size distribution of a dispersion containing ultrafine $TiO_2$ in a dispersion of $TiO_2$-MSSNs for examples 18i-j.

Figure 5. Impact of the particle size of $TiO_2$-MSSN on film formation.

**Detailed description of the invention**

By the term "ultrafine titanium dioxide or $TiO_2$ active compound" in the present invention is meant titanium dioxide material having a crystal size of less than 100 nm, preferably from 3 to 80 nm, more preferably from 8 to 35 nm. This term further means $TiO_2$ particles which are either not coated or coated with organic and/or inorganic coating agents.

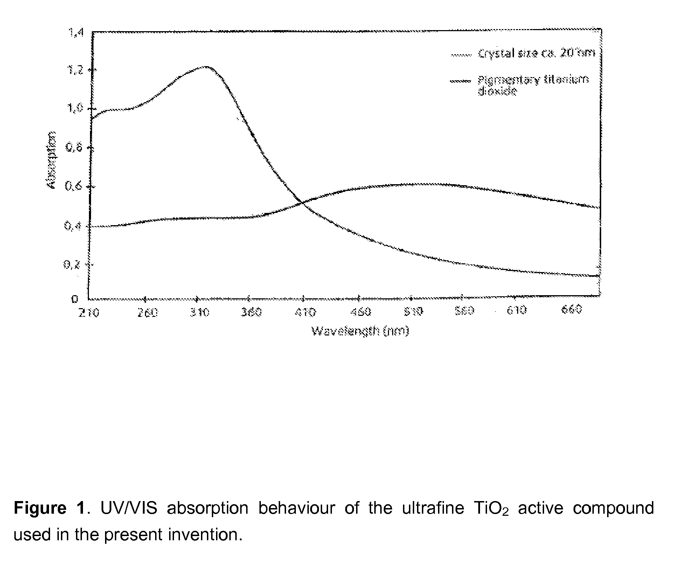

The UV/VIS absorption behaviour of the ultrafine $TiO_2$ active compound is depicted by figure 1 a.

The titanium dioxide in the ultrafine titanium dioxide active compound may have rutile or anatase structure or a mixture thereof, preferably the crystal structure is at least 70% rutile, more preferably at least 99% rutile.

Titanium dioxide active compound according to the invention may include small amounts of other chemicals due to the nature of its manufacturing process such as inorganic and organic surface treatments during its preparation. A surface treatment may include at least one of the following inorganic compounds, aluminium compound, zirconium coumpound, silicon containing inorganic compound, sulphuric acid or organic additive. The resudues comprise preferably coating agents such as aluminum, silicon and/or zirconium oxide hydrates or oxides. The amount of the coating layer is preferably from 0 to 10% by weight of aluminum oxide, from 0 to 10% by weight silicon oxide and from 0 to 5% by weight zirconium oxide calculated as zirconium. In addition, the $TiO_2$ particles may be treated with organic auxiliary agents. Consequently, the ultrafine $TiO_2$ active compound comprises preferably high molecular fatty acids and salts thereof, organic silicon compounds, alkoholes, polyalcohols and amines, most preferably dimethyl polysiloxane, trimethylolethane or polyethylene glycol. The amount of these chemicals is typically below 1 % by weight, preferably from 0.1 % to 0.5% by weight.

Some of the preferred ultrafine $TiO_2$ active compounds are listed in table 1 with their typical characteristics and properties.

**Table 1**

| | M170 | M160 | M212 | M262 | M263 | M111 | M195 | M265 | M765 |
|---|---|---|---|---|---|---|---|---|---|
| Appearance | white powder | white powder | white powder | white powder | white powder | white powder | white powder | white powder | white powder |
| Crystal size nm (ca.) | 14 | 17 | 20 | 20 | 20 | 14 | 15 | 20 | 60 |
| Surface area $m_2/g$ (ca.) | 80 | 70 | 60 | 60 | 60 | 100 | | | |
| Crystal structure | rutile | rutile | rutile | rutile | rutile | rutile | rutile | rutile | rutile |
| $TiO_2$ content % (min) | 75 | 77 | 85 | 85 | 85 | 85 | | | |
| Inorganic treatment | alumina | alumina | alumina | alumina | alumin.-phosphate | alumina | silica | alumina | |
| Organic treatment | silicone | stearic acid | Glycerine | silicone | polyvinyl-pyrrolidon | - | Methicone | silane | silane |
| Property of surface | hydrophobic | hydrophobic | hydrophilic | hydrophobic | hydrophobic | hydrophilic | hydrophobic | hydrophobic | hydrophobic |

The most preferred ultrafine $TiO_2$ active compounds are UV-Titan M195, M212, M262, M265, M040, M290, and M765, the trade names of which refer to ultrafine $TiO_2$ active compounds with additional surface compounds due to surface treatments. These compounds include alumina, glycerol, silica, methicone, dimethicone and triethoxy caprylyl silane and are produced by Kemira Pigments OY. The most preferred ultrafine $TiO_2$ active compound includes Parsol TX from DSM.

According to a preferred embodiment the ultrafine $TiO_2$ according to the invention is prepared by the method described in the applicants earlier patent EP444798.

The details of the MSSN structure, the construction of these structures, properties thereof and equipment required in their manufacturing are described in more detail in the applicant's earlier applications WO2004082666 and WO2007036240. The characteristics and advantages found for these structures and described therein have also been found to apply to the presently described ultrafine $TiO_2$ containing MSSNs.

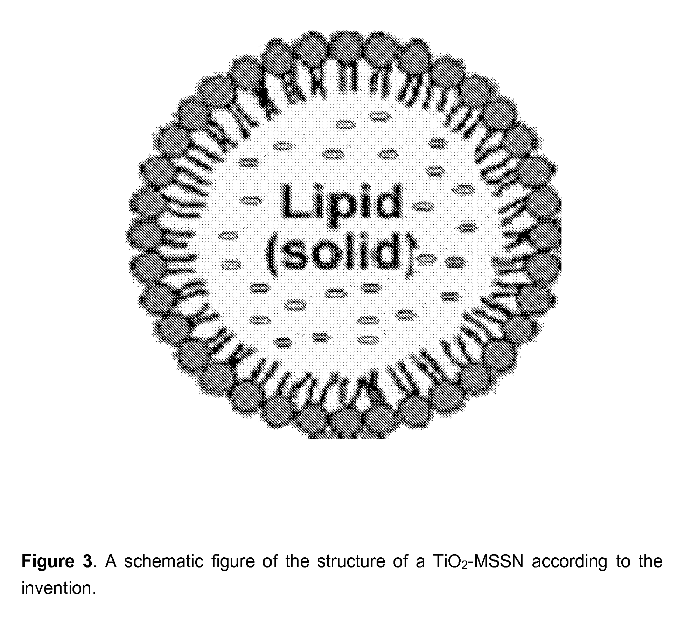

In the first aspect the present invention provides membrane-structured solid nanoparticles (MSSNs) comprising ultrafine titanium dioxide active compound as an active compound therein. In these structures the ultrafine titanium dioxide active compound powder particles are embedded inside the membranes and surrounded by emulsifiers. A schematic figure of a possible structure of $TiO_2$-MSSN is depicted in figure 3. The manufacturing method of these nanoparticles ensure that also slow surfactants may be used and that the surface concentration of the surfactant may be maximised. Due to their manufacturing method which is described below, they have a combination of ultrafine $TiO_2$ active compound vehicle particles and emulsifiers as such to form membranes which infiltrate the entire nanoparticles so that there are emulsifiers in the interior and on the surface of the nanoparticles.

The MSSN structure provides an ideal matrix or network for stabilising the ultrafine $TiO_2$ active compound particles. The tendency of the ultrafine $TiO_2$ particles to agglomerate is considerably reduced when incorporated into this kind of structure with a selection of media provided by the invention as emulsifiers and vehicles. It is especially important for the product application properties that the particle or agglomerate particle size residing inside a single MSSN cell is small enough. It is equally important that the MSSNs used for constructing a dispersion show good $TiO_2$ distribution uniformity and good stability.

The properties of the MSSNs may be measured indirectly by their implementation into dispersion. When in a dispersion the $TiO_2$ active compounds are uniformly distributed inside the MSSNs structures and exhibit a monomodular particle size distribution.

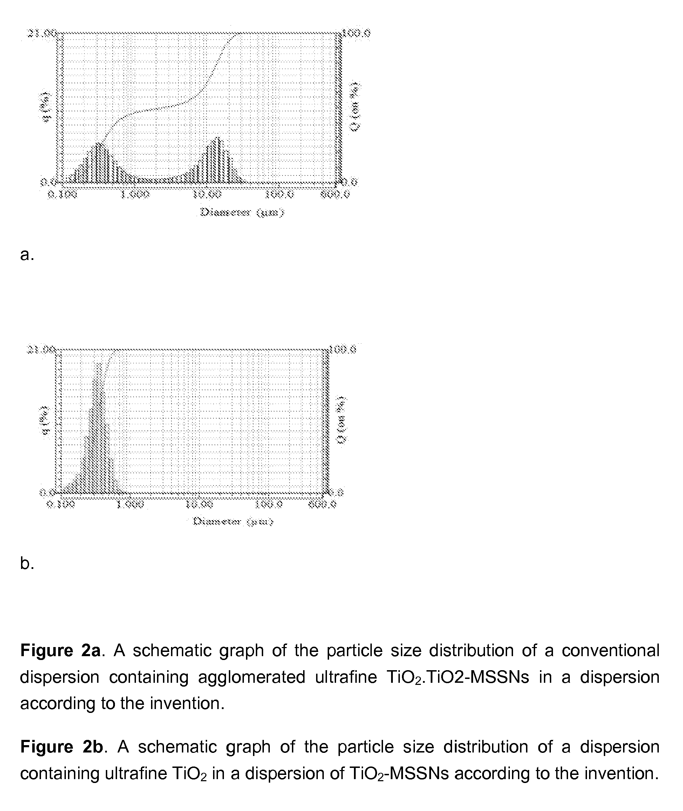

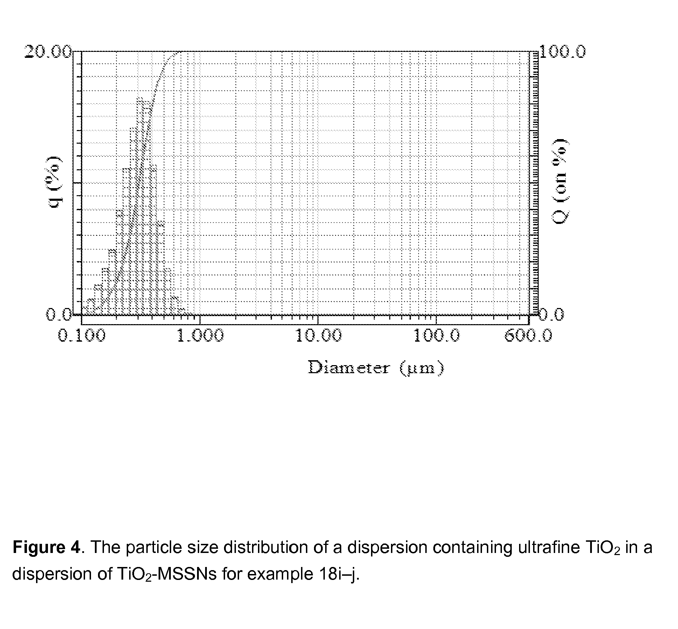

The observed reduced tendency of ultrafine $TiO_2$ particles to agglomerate is verified by measuring the particle size distribution which is shown schematically in figure 2b and for a preferred embodiment in figure 4. The $TiO_2$-MSSN dispersion according to the invention shows a single or monomodular particle size distribution feature when measured with Laser Diffraction Particle Size Analyzer (Horiba LA-300). The average particle diameter $D_{50}$ thereof is from 100 to 800 nm, preferably from 280 to 400 nm. Most preferably the width of the distribution thereof is $\pm$ 150 nm or less, and essentially all particles may be below 1 $\mu$m as depicted by figure 2b. The quality of $TiO_2$, the types of emulsifiers and the parameters of the dispersing method used influence the average particle diameter range and the width of the distribution. Agglomerated particles or an unstable gel typically lead to a bi-modular or even multimodular distribution pattern as schematically shown in figure 2a.

In a preferred embodiment the $TiO_2$-MSSNs have a particle distribution which is $\pm$ 150 nm with no tailing towards the bigger particles.

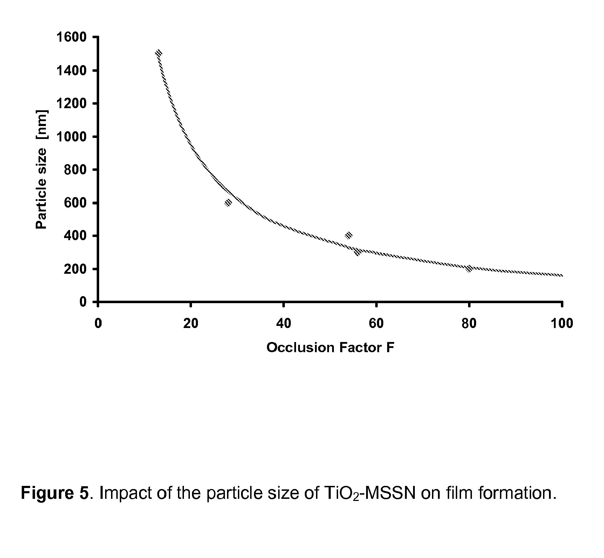

The impact of particle size on film formation is important. Film formation properties may be described by the occlusion factor F. This factor is calculated according to formula 1,

$$F=100*((A-B/A) \tag{1}$$

wherein A is the water loss without sample (=reference) and B is water loss with sample. The higher the occlusion factor is the more coherent the film formation. An occlusion of F=100 correlates to complete film formation. As shown in figure 5 the lower the particle size the better film formation and i.e. a higher occlusion factor is obtained.

The figure 5 further shows that it is not preferred to use particle sizes below 200 nm as the film formation is not further enhanced. The applicable particle size is from 100 nm to 800 nm, preferably from 200 nm to 600 nm, more preferably from 200 nm to 400 nm, most preferably from 280 to 400 nm. The particle size is desired not to be too small due to the possible product safety issues which have been extensively discussed in the field of nanotechnology.

In addition to ultrafine $TiO_2$ active compound the MSSNs of the present invention may include other active components suitable for use therein and required by the desired applications. These compounds include e.g. ultraviolet light absorbers such as those listed in WO2004082666 and in International Cosmetic Ingredient Dictionary and Handbook, Functions, p. 2881. Preferably these other active components are ZnO and organic absorbers such as octocrylene, ethylhexyl methoxycinnamate, butyl methoxydibenzoylmethane, terephthalylidene dicamphor sulfonic acid, bis-ethylhexyloxyphenol methoxyphenyl triazine, ethylhexyl triazone, homosalate, ethylhexyl salicylate, diethylhexyl butamido triazone, sodium phenylbenzimidazole sulfonate.

In the second aspect the present invention provides a method for producing a dispersion containing MSSNs comprising the steps of

a. mixing said ultrafine titanium dioxide active compound with the wax-, polymer-or lipid-based ultrafine titanium dioxide active compound vehicle and at least one emulsifier at a temperature above the melting point or softening point of said ultrafine titanium dioxide active compound vehicle, in order to form phase B, and

b. mechanically mixing said phase B with an aqueous phase A, which optionally comprises an emulsifier, at a temperature above the melting point or softening point of said ultrafine titanium oxide active compound vehicle, without high-pressure homogenization, leading to formation of a lyotropic liquid-crystalline mixed phase A+B, and

c. diluting said mixed phase A+B with an aqueous phase C, which optionally comprises an emulsifier, using stirring but not high-pressure homogenization, to achieve a desired final concentration of said dispersion.

First, ultrafine titanium dioxide active compound is mixed with at least one ultrafine $TiO_2$ active compound vehicle compound. This vehicle comprises agents which are solid or liquid in ambient temperature, oily or waxy compounds, such as oil-, wax-, polymer- or lipid-based compounds.

Secondly, at least one emulsifier is added in parallel to the vechicle compound(s). Examples of suitable emulsifier are listed in WO2004082666.

Examples of suitable oil-, wax-, polymer- or lipid-based compounds are listed in WO2004082666. More preferably these vechicle compounds are selected from the group of particles based on diglycerides, triglycerides, fatty alcohols, their esters or ethers, waxes, lipid peptides or mixtures thereof.

In a preferred embodiment a liquid oily compound is used as the vehicle. In this case the vehicle is liquid at ambient temperature. A gel type of liquid-liquid $TiO_2$ dispersion results after mixing which shows good stability, has a small particle size and which is easily dispersable. This small particle size is the key to enhanced SPF efficacy and film formation. This gel is also very easy to use. It can be incorporated into various emulsions, for example, in commercial O/W- and W/O-lotions by mere stirring. Any emulsifiers may be used in connection with these structures, no negative interactions causing possible dispersion break downs have been observed. This type of gel can be used as such or as a pre-dispersion or dispersion concentrate for different applications such as sun lotions, sun sprays or the like. It is also possible to use this dispersion as such for sun protection due to it's transparency. It has good UV shielding properties and very acceptable skin feeling due to good combatibility with dermatologically preferred surfactants. Furthermore, in this way it is possible to use hydrofobic $TiO_2$ grades in O/W emulsions which is typically very difficult.

The liquid oily compound vehicle comprises preferably organic absorbers such as octocrylene, butyl methoxydibenzoylmethane, octyl methoxycinnamate and the like, dispersants such as PVP/hexadecene copolymer, hexaglyceryl polyricinoleate, polyhydroxystearic acid.

More preferably, the liquid oily compound vehicle is selected from any combination of the compounds of the group consisting of dimethicone, PVP/hexadecene copolymer, heptamethylnonane, behenyl alcohol, glyceryl stearate with or without citrate, disodium ethylene dicocamide PEG-15 disulfate, C12-C15 alkyl benzoate, methoxy cinnamidopropyl hydroxysultaine, hexaglyceryl polyricinoleate, trilaureth-4 phosphate, polyhydroxystearic acid, C11-C13 isoparaffin, polyglyceryl-3 diisostearate, caprylic/capric triglyceride, octocrylene, butyl methoxydibenzoylmethane, octyl methoxycinnamate, petrolatum. The most preferred ones are silicones such as dimethicone.

In an other preferred embodiment a solid waxy compound is used as the vehicle. In this case the vehicle is solid at ambient temperature but it is heated into softening point or melting point i.e. forms a liquid during manufacture. A capsule type of solid-liquid $TiO_2$ dispersion results at ambient temperature which has good stability and which is easily dispersable into hydrophobic systems. This dispersion is similarly easy to use in accordance with the gel. It can be incorporated into various commercial O/W-lotions. It can be used as a pre-dispersion or dispersion concentrate for different applications such as sun lotions, sun srays or the like. It is also possible to use this dispersion as such. It has good UV shielding properties and very acceptable skin feeling.

Preferably, the solid waxy compound vehicle comprises at least one cosmetically approved wax. More preferably the solid waxy compound vehicle selected from any combination of the compounds of the group consisting of bees wax, cetyl palmitate, microstalline paraffin wax, paraffin wax, carnauba wax, candelilla wax.

The mixing of ultrafine $TiO_2$ active compound vehicle and emulsifier is performed at a temperature above the melting point or softening point of said ultrafine titanium dioxide active compound vehicle thus forming a phase B. For the gel type liquid-liquid $TiO_2$ dispersion ambient temperature is high enough to keep the vehicle component liquid, but an elevated temperature is required for the solid-liquid $TiO_2$ dispersion. Preferably the temperature is 5°C above the melting or the softening point of the vehicle material.

The average diameter of ultrafine titanium dioxide active compound vehicle particles and/or droplets at ambient temperature are preferably from 50 to 1000 nm.

In addition, it is possible to use dispersion stabilizers. They can be used in amounts of 0.01% to 20% by weight, preferably from 0.05% to 5% by weight. Examples of suitable substances are described e.g. in WO2004082666.

In the next step the formed mixture, phase B, is mechanically mixed with an aqueous phase A. It is possible to use water, aqueous solutions or mixtures of water with water-miscible liquids such as glycerol or polyethylene glycol as the aqueous phase A. Most preferably phase A comprises in addition to water laureth-23, steareth-100, sodium lauroyl lactylate, disodium ethylene dicocamide PEG-15 disulfate, disodium EDTA dihydrate, capryl glucoside, Glycerine, PEG-25-PPG-30 phosphate, sodium laureth sulfate, lactic acid, PEG-100 stearate, lauryl glucoside, decyl glucoside, caprylyl/capryl glucoside, poloxamer 407, ceteareth-50.

Further, additional components for the aqueous phase A may comprise for example, mannose, glucose, fructose, xylose, trehalose, mannitol, sorbitol, xylitol or other polyols such as polyethylene glycol and also electrolytes such as sodium chloride. These additional components may be used in an amount of 1% to 60% by weight, preferably from 1% to 30% by weight, based on the aqueous phase A. The weight ratio of phase B to phase A is preferably from 1:5 to 5:1, more preferably from 1:2 to 2:1, most preferably from 1:1.5 to 1.5:1.

Mixing in steps a and b may be achieved by means of conventional, mechanical agitators which have the agitation performance comparable to that of a household mixer (or household kitchen mixer). In laboratory operation, for example, it is possible to achieve sufficient agitation using low power mixers such as about 300 W, for example a Braun kitchen mixer. A clear advantage in the present method is that no high energy or high pressure homogenization equipment or mixing is required. A reasonable mixing according to the present invention provides low energy, lamellar flow at ambient pressure which facilitates the use of slow surfactants and enables suitable droplet formation and size uniformity. During mixing the $TiO_2$-MSSN phase formation becomes evident and detectable as the viscosity of the solution reaches at a certain point a maximum and starts to decrease rapidly.

Optionally, a further emulsifier is added to phase A. Examples of suitable emulsifiers are described e.g. in WO2004082666. The most preferred emulsifiers are laureth-23, steareth-100, sodium lauroyl lactylate, disodium ethylene dicocamide PEG-15 disulfate, capryl/caprylyl glucoside, Glycerine, PEG-25-PPG-30 phosphate, sodium laureth sulfate, lactic acid, PEG-100 stearate, lauryl glucoside, decyl glucoside, poloxamer 407, ceteareth-50.

The mixture of phase A and B is formed at a temperature above the melting or softening point of said active compound vehicle similarly to mixing of the ultrafine $TiO_2$ active compound vehicle and emulsifier. No high-pressure homogenization is required. As a result, a lyotropic liquid-crystalline mixed phase comprising ultrafine $TiO_2$-MSSNs is obtained. In these particles the combination of active compound vehicles and emulsifiers form membranes which infiltrate the entire nanoparticles so that there are emulsifiers in the interior and on the surface of the formed nanoparticles enabling a stable and finely divided ultrafine $TiO_2$ containing product.

Furthermore, suitable additives such as feel enhancers, dispersants, and the like. may be used for enriching the properties of the dispersion described in more detail in WO2004082666.

The mixed phase A+B is further mixed with an aqueous phase C at ambient temperature for providing the gel type liquid-liquid $TiO_2$ dispersion or below the melting or softening point of said active compound vehicle for providing the capsule-type solid-liquid $TiO_2$ dispersion. Preferably for the solid-liquid $TiO_2$ dispersion the temperature is at least 5°C below the melting or softening point of the active compound vehicle, more preferably at least 15°C below, most preferably from 30 to 40°C below. Moderate stirring is applied using equipment and techniques known in the art, preferably stirring is performed ensuring that no cavitations are formed. Preferably the product will be self emulsifying if phase C is water. Thus, no high-pressure homogenization is required or necessary.

The aqueous phase C comprises mainly water. Optionally, at least one thickener is added to phase C or the mixture of phase C and the mixed phase A+B by mixing. Preferable thickeners comprise carbomers, xanthan gum, mixtures thereof. Phase C may further comprise a preservative, preferably phenoxyethanol, methylparaben, ethylparaben, butylparaben, propylparaben, isobutylparaben or ethanol, and/or sequestrant such as EDTA.

The mechanical mixing of phases A and B and the stirring of phase C take place preferably with agitators which have a peripheral speed in the range from 1 to 20 m/s, more preferably 1 to 3 m/s. The shearing action of the agitator corresponds preferably to the shearing action of a commercial household kitchen mixer.

In the third aspect the present invention provides an aqueous ultrafine $TiO_2$-MSSN dispersion obtained by the above described method.

The amount of vehicle, based on the total aqueous $TiO_2$ vehicle dispersion, is for gel type dispersion preferably from 0.1% to 70% by weight, more preferably from 1% to 60% by weight, for example, from 0.1% to 30% or from 1% to 10% by weight.

The amount of $TiO_2$ to be incorporated into the MSSN structure is dependent on the type and amount of $TiO_2$ used and on the type of vehicle used. For example, the product is more thixotropic if the $TiO_2$ particle is coated with a silicon compound. Moreover, the smaller the crystal size i.e. the larger the specific surface area of $TiO_2$ the more difficult

it is the be dispersed. Furthermore, non-polar vehicles are better in incorporating the $TiO_2$ particles than polar ones, and naturally the concentration of $TiO_2$ is a critical factor.

The amount of ultrafine $TiO_2$ active compound in the aqueous $TiO_2$-MSSN dispersion based on the total amount of aqueous $TiO_2$ vehicle dispersion, is for the gel-type liquid-liquid $TiO_2$ dispersion from 0.1% to 50%, preferably from 0.1% to 40% by weight, more preferably from 1% to 40% by weight, most preferably from 1% to 35% such as from 1 % to 30% by weight.

The amount of ultrafine $TiO_2$ active compound in the aqueous $TiO_2$-MSSN dispersion based on the total amount of aqueous $TiO_2$ vehicle dispersion, is for the capsule type solid-liquid $TiO_2$ dispersion from 0.1% to 50%, preferably from 0.1% to 30% by weight, more preferably from 1% to 20% by weight, most preferably from 1 % to 10% by weight.

The amount of ultrafine $TiO_2$ possible to incorporate into these dispersions depends on the chosen vehicle compounds. The final desired concentration of $TiO_2$ in the application such as cosmetic formulation, determines the typical concentration in the disperson. The reduced amount of aggromerated $TiO_2$ enhances the efficacy of the $TiO_2$ amount incorporated in the dispersion.

The amount of emulsifier(s) in the aqueous $TiO_2$ vehicle dispersion is at least 1% by weight, preferably from 1 to 60% by weight.

The dispersion provided by the present invention is readily usable. It can be easily incorporated into various types of application such as in sun screen lotions or emulsion. Moreover, it is possible to combine this dispersion with various emulsion, salve or cream ingredients. The material incompatibility issues are reduced compared to conventional dispersions.

The ultrafine $TiO_2$-MSSN dispersions of the invention are stable in storage and even with a higher nanoparticle concentration enjoy very good fluidity which enhances and facilitates its use in final application formulas.

Dispersions according to the invention are stable for at least 1 year, preferably at least 6 months, most preferably at least 3 months.

Further aspects of the present invention provide different uses of the MSSNs of any of the claims 1-6 or the aqueous dispersion of any of the claims 7-11 for O/W or W/O dispersions in general, especially in cosmetics and coatings. Preferably these dispersions are used in daycare or suncare products such as lotions and sprays and especially in products wherein high SPF efficacy or UV-protection is required.

In a further embodiment of the present invention the $TiO_2$ active compound incorporated into the MSSN structure may be photocatalytically active and the final dispersion may be used in any application wherein photocatalytic properties, good dispersing properties and/or transparency are essential such as various coatings.

In addition to the properties described above, the same advantages and merits apply to the ultrafine $TiO_2$-MSSN products, to the dispersion comprising it and to the method for its preparation and applications derived therefrom as already described in W02004082666.

A skilled person in the field is able to optimize the method according to the chosen raw materials used.

The invention is elucidated in more detail by the following examples.

**Examples**

In the examples below the following compounds listed in table 2 are employed:

**Table 2**

| Trade name | Manufacturer | CTFA/INCI |
|---|---|---|
| Abil 100 | goldschmidt | Dimethicone |
| Antaron V-216 | ISP | PVP/hexadecene Copolymer |
| Arlamol HD | Uniqema | Heptamethylnonane |
| Beeswax Care 144 | Paramelt | Beeswax |
| Brij 35 | Uniqema | Laureth-23 |
| Brij 700 | Uniqema | Steareth-100 |
| Ceralution F | Sasol | Sodium Lauroyl Lactylate (and) Disodium Ethylene Dicocamide PEG-15 Disulfate |
| Ceralution H | Sasol | Behenyl Alcohol (and) Glyceryl Stearate (and) Glyceryl Stearate/citrate (and) Disodium Ethylene Dicocamide PEG-15 Disulfate |

(continued)

| Trade name | Manufacturer | CTFA/INCI |
|---|---|---|
| Cutina CP | Cognis | Cetyl Palwithate |
| Dekaben C | Jan Dekker | Phenoxyethanol (and) Methylparaben (and) Ethylparaben (and) Butylparaben (and) Propylparaben (and) Isobutylparaben |
| Demin. water | - | Water |
| Edeta BD | BASF | Disodium EDTA Dihydrate |
| Finsolv TN | Finetex | C12-15 Alkyl Benzoate |
| Galaxy-Sunbeat | Galaxy Surfactant Ltd. | Methoxy Cinnamidopropyl Hydroxysultaine |
| Glucopon 215 CSUP | Cognis | Capryl Glucoside |
| Glycerine | Uniqema | Glycerine |
| Hexaglyn PR-15 | Nikko | Hexaglyceryl Polyricinoleate |
| Hostaphat KL 340 D | Clariant | Trilaureth-4 Phosphate |
| Innosperse DSP-OL 300 | Innospec Liwithed | Polyhydroxystearic acid |
| Innosperse DSP-W90 | Innospec Liwithed | PEG-25-PPG-30 Phosphate |
| Isopar L | Exxon | C11-13 Isoparaffin |
| Keltrol | Kelco | Xanthan Gum |
| Lameform TGI | Cognis | Polyglyceryl-3 Diisostearate |
| Marlinat 242/70 | Sasol | Sodium Laureth Sulfate |
| Miglyol 812 N | Sasol | Caprylic/Capric Triglyceride |
| Milchsäure (90%) | Merck | Lactic Acid |
| Myrj 59P Pharma | Uniqema | PEG-100 Stearate |
| Neo Heliopan 303 | Cosnaderm | Octocrylene |
| Paracera M | Paramelt | Microstalline Wax |
| Parsol 1789 | DSM | Butyl Methoxydibenzoylmethane |
| Parsol MCX | DSM | Octyl Methoxycinnamate |
| Parsol TX | DSM | Titanium Dioxide (and) Silica (and) Dimethicone |
| Plantacare 1200 UP | Cognis | Lauryl Glucoside |
| Plantacare 2000 UP | Cognis | Decyl Glucoside |
| Plantacare 810 UP | Cognis | Caprylyl / Capryl Glucoside |
| Pluronic F-127 | BASF | Poloxamer 407 |
| Polarit GZN | Tromm | Paraffin Wax |
| UV-Titan M 160 | Kemira | Titanium Dioxide (and) Alumina (and) Glycerol |
| UV-Titan M 161 | Kemira | Titanium Dioxide (and) Alumina (and) Stearic Acid |
| UV-Titan M 170 | Kemira | Titanium Dioxide (and) Alumina (and) Methicone |
| UV-Titan M 195 | Kemira | Titanium Dioxide (and) Silica (and) Methicone |
| UV-Titan M 212 | Kemira | Titanium Dioxide (and) Alumina (and) Glycerol |
| UV-Titan M 262 | Kemira | Titanium Dioxide (and) Alumina (and) Dimethicone |
| UV-Titan M 263 | Kemira | Titanium Doxide (and) Alumina (and) Sodium Hexametaphosphate (and) Poyvinylpyrrolidone |

(continued)

| Trade name | Manufacturer | CTFA/INCI |
|---|---|---|
| UV-Titan M 265 | Kemira | Titanium Dioxide (and) Alumina (and) Silane |
| UV-Titan X 040 | Kemira | Titanium Dioxide (and) Silica (and) Silane |
| UV-Titan X 205 | Kemira | Titanium Dioxide |
| UV-Titan X 290 | Kemira | |
| UV-Titan X 765 | Kemira | Titanium Dioxide (and) Alumina (and) Silane |
| Wacker Belsil DM 1 plus | Wacker Chemie | Dimethicone |
| Wacker Belsil DM 100 | Wacker Chemie | Dimethicone |
| Wacker Belsil DM 12500 | Wacker Chemie | Dimethicone |
| Wacker Belsil DM 60000 | Wacker Chemie | Dimethicone |
| Vaseline weiss | Mainopharm GmbH | Petrolatum |
| Volpo CS 50 | Croda | Ceteareth-50 |
| Z-Cote | BASF | Zinc Oxide |
| Z-Cote HP 1 | BASF | Zinc Oxide (and) Triethoxycaprylsilane |

The aqueous ultrafine $TiO_2$ vehicle dispersion is produced by separately heating phases A and B, described below, to 60°C. Phase B is then stirred into phase A, and using a Braun kitchen mixer (maximum power 350 W) with an agitating-blade diameter of 50 mm. The mixture is homogenized until the droplet size is small enough. The evolution of particle size is followed by measuring the gel viscosity. The optimum (target) viscosity depends on the formulation. In formulas where phase C is needed it is added at ambient temperature, mixing likewise at room temperature, to the hot emulsion. Agitation is again carried out with a Braun kitchen mixer.

The quality of the dispersion is assessed based on the particle size analyses (PSA) with Horiba LA-300 particle size analyser: the average particle diameter, standard deviation, weight fraction of particles having a diameter of less than 1 $\mu$m, and the specific surface area (cm$^2$/cm$^3$) are determined.

The resulting good quality dispersions have an average particle size from 280 nm to 400 nm, standard deviation below 0,15 $\mu$m and weight fraction of particles having a diameter of less than 1 $\mu$m is 100%.

The compositions of the individual phase and the stated parameters are evident from the table 3.

## Table 3

| Example | 1a | 1b | 1c | 1d |
|---|---|---|---|---|
| | [wt-%] | [wt-%] | [wt-%] | [wt-%] |
| **Phase A** | | | | |
| Ceralution F | 4.3 | 5.7 | 5.5 | 3.22 |
| Plantacare 1200 UP | 17.1 | 23.0 | 21.9 | 12.87 |
| Glycerinee | 17.1 | 23.0 | 21.9 | 12.87 |
| **Phase A1** | | | | |
| UV-TITAN M 212 | 3.4 | 16.5 | 15.7 | 9.25 |
| Demin. Water | 4.2 | 20.4 | 19.4 | 11.44 |
| **Phase B** | | | | |
| Abil 100 | 51.0 | 10.8 | 15.6 | 9.18 |
| Ceralution H | 2.9 | 0.6 | | |
| **Phase C** | | | | |
| Demin. water | | | | 41.18 |
| **Sum** | 100.0 | 100.0 | 100.0 | 100.00 |

| Example | 2a | 2b | 2c | 2d | 2e | 2f |
|---|---|---|---|---|---|---|
| | [wt-%] | [wt-%] | [wt-%] | [wt-%] | [wt-%] | [wt-%] |
| **Phase A** | | | | | | |
| Ceralution F | 5.3 | 4.7 | 3.1 | 2.3 | 1.8 | 0.9 |
| Plantacare 1200 UP | 21.3 | 18.9 | 12.6 | 9.3 | 7.4 | 3.7 |
| Glycerinee | 21.3 | 18.9 | 12.6 | 9.3 | 7.4 | 3.7 |
| **Phase B** | | | | | | |
| Ceralution H | | 5.2 | 1.6 | 1.7 | 1.8 | 0.9 |
| Abil 100 | 36.5 | 36.6 | 54.3 | 60.0 | 63.3 | 31.6 |
| UV-TITAN M 160 | 15.6 | 15.7 | 15.7 | 17.4 | 18.3 | 9.2 |
| **Phase C** | | | | | | |
| Demin. water | | | | | | 50.0 |
| **Sum** | 100.0 | 100.0 | 100.0 | 100.0 | 100.0 | 100.0 |

| Example | 3a | 3b | 3c | 3d | 3e | 3f | 3g | 3h | 3i |
|---|---|---|---|---|---|---|---|---|---|
| | [wt-%] | [wt-%] | [wt-%] | [wt-%] | [wt-%] | [wt-%] | [wt-%] | [wt-%] | [wt-%] |
| **Phase A** | | | | | | | | | |
| Ceralution F | 4.9 | 5.0 | 5.0 | 4.7 | 4.1 | 4.7 | 3.8 | 4.0 | 4.0 |
| Plantacare 1200 UP | 19.8 | 20.1 | 19.9 | 18.7 | 16.5 | 18.8 | 15.1 | 16.1 | 16.1 |
| Glycerinee | 19.8 | 20.1 | 19.9 | 18.7 | 16.5 | 18.8 | 15.1 | 16.1 | 16.1 |
| **Phase B** | | | | | | | | | |
| Ceralution H | 1.1 | 1.1 | 1.1 | 1.1 | 1.2 | 1.1 | 1.3 | 1.2 | 1.2 |
| Abil 100 | 38.1 | 37.6 | 31.5 | | | | | | |
| Hostaphat KL 340 D | | | 6.3 | | | | | | |
| Finsolv TN | | | | 39.7 | 43.2 | 39.7 | 41.2 | 39.8 | 39.8 |
| Parsol 1789 | | | | | | | 4.1 | 4.0 | 4.0 |
| UV-TITAN X195 | 16.3 | | | | | 17.0 | | | 18.7 |
| UV-TITAN M 212 | | 16.1 | 16.2 | | | | | | |
| UV-TITAN M 160 | | | | 17.0 | | | 19.4 | | |
| Parsol TX | | | | | 18.5 | | | 18.7 | |
| **Sum** | 100.0 | 100.0 | 100.0 | 100.0 | 100.0 | 100.0 | 100.0 | 100.0 | 100.0 |

| Example | 4a | 4b | 4c | 4d | 4e | 4f | 4g | 4h | 4i | 4j |
|---|---|---|---|---|---|---|---|---|---|---|
| | [wt-%] | [wt-%] | [wt-%] | [wt-%] | [wt-%] | [wt-%] | [wt-%] | [wt-%] | [wt-%] | [wt-%] |
| **Phase A** | | | | | | | | | | |
| Ceralution F | 5.5 | 5.7 | 4.6 | 4.8 | 4.9 | 5.7 | 5.1 | 5.6 | 5.6 | 6.0 |
| Plantacare 1200 UP | 21.8 | 22.9 | 18.5 | 19.2 | 19.5 | 22.9 | 20.6 | 22.3 | 22.5 | 23.8 |
| Glycerine | 21.8 | 22.9 | 18.5 | 19.2 | 19.5 | 22.9 | 20.6 | 22.3 | 22.5 | 23.8 |
| **Phase B** | | | | | | | | | | |
| Ceralution H | 1.0 | 0.9 | 1.1 | 1.1 | 1.1 | 1.0 | 1.1 | 1.0 | 1.0 | 0.9 |
| Wacker Belsil DM 100 | 34.9 | 33.2 | 34.4 | 27.9 | 38.6 | 33.3 | 36.8 | 34.2 | 33.8 | |
| Abil 100 | | | | | | | | | | 31.8 |
| Parsol TX | 15.0 | | | | | | | | | |
| UV-Titan M 160 | | 14.2 | 22.9 | 27.9 | | | | | | 13.6 |
| UV-Titan X 040 | | | | | 16.5 | | | | | |
| UV-Titan X 765 | | | | | | 14.3 | | | | |
| UV-Titan M 262 | | | | | | | 15.8 | | | |
| UV-Titan X265 | | | | | | | | 14.7 | | |
| Z-Cote HP 1 | | | | | | | | | 14.5 | |
| **Sum** | 100.0 | 100.0 | 100.0 | 100.0 | 100.0 | 100.0 | 100.0 | 100.0 | 100.0 | 100.0 |

| Example | 5a | 5b | 5c | 5d | 5e | 5f | 5g | 5h | 5i | 5j | 5k | 5l |
|---|---|---|---|---|---|---|---|---|---|---|---|---|
| | [wt-%] | [wt-%] | [wt-%] | [wt-%] | [wt-%] | [wt-%] | [wt-%] | [wt-%] | [wt-%] | [wt-%] | [wt-%] | [wt-%] |
| **Phase A** | | | | | | | | | | | | |
| Ceralution F | 3.3 | 3.1 | 2.8 | 4.2 | 4.5 | 4.4 | 3.5 | 3.4 | 2.7 | 3.5 | 2.7 | 4.3 |
| Plantacare 1200 UP | 13.0 | 12.3 | 11.1 | 16.9 | 17.8 | 17.6 | 13.9 | 13.6 | 10.9 | 14.0 | 11.0 | 17.1 |
| Glycerine | 13.0 | 12.3 | 11.1 | 16.9 | 17.8 | 17.6 | 13.9 | 13.6 | 10.9 | 14.0 | 11.0 | 17.1 |
| | | | | | | | | | | | | |
| **Phase B** | | | | | | | | | | | | |
| Ceralution H | 1.4 | 1.4 | 1.5 | 1.2 | 1.2 | 1.2 | 1.3 | 1.4 | 1.5 | 1.3 | 1.5 | 1.2 |
| Miglyol 812NN | 48.5 | | | | | | | | | | | |
| Vaseline weiss | | 49.6 | | | | | 40.4 | 34.0 | | | | |
| Isopar L | | | 51.5 | | | | | | 44.4 | 33.6 | 29.5 | |
| Wacker Belsil DM 100 | | | | | 42.5 | | | | | | | |
| Wacker Belsil DM 12500 | | | | | 41.1 | | | | | | | |
| Wacker Belsil DM 60000 | | | | | | 41.5 | | | | | | |
| Neo Heliopan 303 | | | | | | | | | | | | 42.3 |
| UV-Titan M 160 | 20.8 | 21.2 | 22.1 | 18.2 | 17.6 | 17.8 | 26.9 | 34.0 | 29.6 | 33.6 | 44.3 | 18.1 |
| | | | | | | | | | | | | |
| **Sum** | 100.0 | 100.0 | 100.0 | 100.0 | 100.0 | 100.0 | 100.0 | 100.0 | 100.0 | 100.0 | 100.0 | 100.0 |

| Example | 6a | 6b | 6c | 6d | 6e | 6f | 6g |
|---|---|---|---|---|---|---|---|
| | [wt-%] | [wt-%] | [wt-%] | [wt-%] | [wt-%] | [wt-%] | [wt-%] |
| **Phase A** | | | | | | | |
| Ceralution F | 5.6 | 5.1 | 4.7 | 3.7 | 6.3 | 5.5 | 5.4 |
| Plantacare 1200 UP | 22.3 | 20.6 | 18.6 | 14.7 | 25.4 | 21.9 | 21.7 |
| Glycerine | 22.3 | 20.6 | 18.6 | 14.7 | 25.4 | 21.9 | 21.7 |
| **Phase B** | | | | | | | |
| Ceralution H | 1.0 | 1.1 | 1.1 | 1.3 | 0.8 | 1.0 | 1.0 |
| Wacker Belsil DM 1 plus | 39.0 | 36.8 | 34.2 | 32.8 | | | |
| Neo Heliopan 303 | | | | | | | 35.2 |
| Parsol MCX | | | | | 29.5 | 34.9 | |
| UV-TITAN M 160 | 9.8 | 15.8 | 22.8 | 32.8 | 12.6 | | |
| Parsol TX | | | | | | 14.9 | 15.1 |
| **Sum** | 100.0 | 100.0 | 100.0 | 100.0 | 100.0 | 100.0 | 100.0 |

| Example | 7a | 7b | 7c | 7d | 7f | 7g | 7h | 7i | 7j |
|---|---|---|---|---|---|---|---|---|---|
| | [wt-%] | [wt-%] | [wt-%] | [wt-%] | [wt-%] | [wt-%] | [wt-%] | [wt-%] | [wt-%] |
| **Phase A** | | | | | | | | | |
| Ceralution F | 2.1 | 3.3 | 2.1 | 3.4 | 3.4 | 3.3 | 3.5 | 3.2 | 3.1 |
| Plantacare 1200 UP | 7.4 | 13.0 | 7.4 | | | 13.0 | 12.5 | 11.3 | 10.9 |
| Brij 35 | 1.0 | 1.6 | 1.0 | | | | | | |
| Brij 700 | | | | 5.8 | | 1.6 | 1.7 | 1.5 | 1.5 |
| Glycerine | 8.4 | 11.5 | 7.4 | 12.5 | 12.5 | 11.5 | 14.2 | 12.8 | 12.4 |
| Innosperse DSP-W90 | | | 1.0 | | | | | | |
| Demin. water | | | | 5.8 | 5.8 | | | | |
| Volpo CS50 | | | | 5.8 | | | | | |
| **Phase B** | | | | | | | | | |
| Ceralution H | 4.3 | 3.4 | 4.3 | 3.6 | 3.6 | 3.5 | 3.6 | 3.3 | 3.2 |
| Arlamol HD | 48.1 | 38.2 | 48.1 | 39.5 | 39.5 | 38.1 | 40.4 | 36.5 | 35.4 |
| UV-TITAN M 160 | 28.7 | 29.0 | 28.7 | 29.5 | 29.5 | 29.0 | 24.1 | 21.8 | 21.1 |
| **Phase C** | | | | | | | | | |
| Demin. water | | | | | | | | 9.6 | 12.4 |
| **Sum** | 100.0 | 100.0 | 100.0 | 100.0 | 100.0 | 100.0 | 100.0 | 100.0 | 100.0 |

| Example | 8a | 8b | 9a | 9b | 10a | 10b |
|---|---|---|---|---|---|---|
| | [wt-%] | [wt-%] | [wt-%] | [wt-%] | [wt-%] | [wt-%] |
| **Phase A** | | | | | | |
| Ceralution F | 7.7 | 6.4 | 4.4 | 2.2 | 4.4 | 4.4 |
| Plantacare 1200 UP | 15.4 | 12.8 | | | | |
| Plantacare 2000 UP | | | 17.8 | 8.9 | | |
| Plantacare 810 UP | | | | | 17.8 | |
| Pluronic F-127 | | | | | | 17.8 |
| Glycerine | 15.4 | 12.8 | 17.8 | 8.9 | 17.8 | 17.8 |
| | | | | | | |
| **Phase B** | | | | | | |
| Ceralution H | 2.6 | 2.2 | 2.5 | 1.3 | 2.5 | 2.5 |
| Miglyol 812N | 39.7 | 33.1 | 40.2 | 20.1 | 40.2 | 40.2 |
| Parsol TX | 19.1 | 15.9 | 17.2 | 8.6 | 17.2 | 17.2 |
| Innosperse DSP-OL 300 | 0.2 | 0.1 | | | | |
| | | | | | | |
| **Phase C** | | | | | | |
| Demin. water | | 16.7 | | 50.1 | | |
| | | | | | | |
| **Sum** | 100.0 | 100.0 | 100.0 | 100.0 | 100.0 | 100.0 |

| Example | 11 | 12a | 12b | 12c | 12d |
|---|---|---|---|---|---|
| | [wt-%] | [wt-%] | [wt-%] | [wt-%] | [wt-%] |
| **Phase A** | | | | | |
| Ceralution F | 4.0 | 1.9 | 1.9 | 2.3 | 2.2 |
| Plantacare 1200 UP | 16.0 | 7.4 | 7.4 | 9.1 | 8.7 |
| Glycerine | 16.0 | 7.4 | 7.4 | 9.1 | 8.7 |
| | | | | | |
| **Phase B** | | | | | |
| Ceralution H | 4.8 | 2.8 | 2.8 | 2.5 | 2.4 |
| Miglyol 812N | 39.6 | 43.1 | 43.1 | 38.3 | 36.3 |
| UV-TITAN M160 | 19.0 | 20.8 | | 9.2 | 16.6 |
| Z-cote | | | 20.8 | 9.2 | |
| Galaxy-Sunbeat | | | | | 0.9 |
| Antaron V-216 | 0.6 | | | | |
| | | | | | |
| **Phase C** | | | | | |
| Demin. water | | 16.7 | 16.7 | 20.4 | 24.4 |
| | | | | | |
| **Sum** | 100.0 | 100.0 | 100.0 | 100.0 | 100.0 |

| Example | 13a | 13b | 13c | 14a | 14b | 14c |
|---|---|---|---|---|---|---|
| | [wt-%] | [wt-%] | [wt-%] | [wt-%] | [wt-%] | [wt-%] |
| **Phase A** | | | | | | |
| Ceralution F | 1.8 | 2.9 | 2.9 | 1.8 | 2.9 | 2.9 |
| Plantacare 1200 UP | 7.3 | 11.5 | 11.5 | 7.3 | 11.5 | 11.5 |
| Glycerine | 7.3 | 11.5 | 11.5 | 7.3 | 11.5 | 11.5 |
| Edeta BD | 0.1 | | | 0.1 | | |
| **Phase B** | | | | | | |
| Ceralution H | 3.3 | 2.6 | 2.5 | 3.3 | 2.6 | 2.5 |
| Miglyol 812N | 37.1 | 29.3 | 43.6 | 37.1 | 29.3 | 43.6 |
| UV-TITAN M160 | 27.8 | 29.3 | | 27.8 | 29.3 | |
| Innosperse DSP-OL 300 | 2.0 | 2.0 | | 2.0 | 2.0 | |
| Z-cote | | | 17.2 | | | 17.2 |
| **Phase C** | | | | | | |
| Keltrol | 0.1 | | | 0.1 | | |
| Demin. water | 12.6 | 10.8 | 10.8 | 12.6 | 10.8 | 10.8 |
| Edeta BD | 0.4 | | | 0.4 | | |
| **Sum** | 100.0 | 100.0 | 100.0 | 100.0 | 100.0 | 100.0 |

| Example | 15a | 15b | 15c | 16a | 16b | 17 |
|---|---|---|---|---|---|---|
| | [wt-%] | [wt-%] | [wt-%] | [wt-%] | [wt-%] | [wt-%] |
| **Phase A** | | | | | | |
| Ceralution F | 2.1 | 1.8 | 1.8 | 0.9 | 0.9 | 2.8 |
| Plantacare 1200 UP | 8.4 | 7.3 | 7.3 | 3.8 | 3.5 | 11.0 |
| Glycerine | 8.4 | 7.3 | 7.3 | 3.8 | 3.5 | 11.0 |
| **Phase B** | | | | | | |
| Ceralution H | 3.2 | 2.8 | 2.8 | 3.6 | 3.4 | |
| Miglyol 812N | 51.2 | 44.5 | 44.5 | 57.8 | 53.7 | 45.0 |
| UV-TITAN M160 | 26.7 | 23.2 | 23.2 | 30.1 | 28.0 | 30.2 |
| **Phase C** | | | | | | |
| Keltrol | | | 0.1 | | | |
| Demin. water | | 13.0 | 12.9 | | 7.1 | |
| **Sum:** | 100.0 | 100.0 | 100.0 | 100.0 | 100.0 | 100.0 |

| Example | 18a | 18b | 18c | 18d | 18e | 18f | 18g | 18h | 18i | 18j |
|---|---|---|---|---|---|---|---|---|---|---|
| | [wt-%] | [wt-%] | [wt-%] | [wt-%] | [wt-%] | [wt-%] | [wt-%] | [wt-%] | [wt-%] | [wt-%] |
| **Phase A** | | | | | | | | | | |
| Marlinat 242/70 | 7.6 | 7.8 | 10.7 | 8.7 | 7.1 | 10.6 | 8.1 | 10.0 | 8.5 | 8.7 |
| Myrj 59P Pharma | 3.4 | 3.5 | 4.8 | 3.9 | 3.2 | 4.7 | 3.6 | 4.5 | 3.8 | 3.9 |
| Lactic Acid (90%) | 0.1 | 0.1 | 0.2 | 0.1 | 0.1 | 0.2 | 0.1 | 0.2 | 0.1 | 0.2 |
| Glycerine | 10.5 | 10.8 | 14.7 | 12.0 | 9.8 | 14.6 | 11.1 | 13.9 | 11.8 | 12.0 |
| Demin. water | 3.2 | 3.2 | 4.4 | 3.6 | 2.9 | 4.4 | 3.3 | 4.2 | 3.5 | 3.6 |
| **Phase B** | | | | | | | | | | |
| Cutina CP | 22.1 | 21.9 | 20.4 | | | | | | | |
| Polarit GZN | | | | 21.1 | 22.6 | | | 18.7 | 20.1 | 21.1 |
| Beeswax Care 144 | | | | | | 19.3 | | | | |
| Paracera M | | | | | | | 21.7 | | | |
| Finsolv TN | | | | | | | | 3.7 | | |
| Wacker-Belsil DM 100 | | | | | | | | | 4.0 | |
| Parsol TX | 8.8 | | | 8.4 | | 7.7 | 8.7 | 7.5 | 8.0 | 8.4 |
| UV-TITAN X040 | | 8.8 | 4.1 | | 9.1 | | | | | |
| **Phase C** | | | | | | | | | | |
| Demin. water | 44.2 | 43.9 | 40.8 | 42.2 | 45.3 | 38.6 | 43.4 | 37.4 | 40.1 | 42.1 |
| | | | | | | | | | | |
| **Sum:** | 100.0 | 100.0 | 100.0 | 100.0 | 100.0 | 100.0 | 100.0 | 100.0 | 100.0 | 100.0 |

| Example | 19a | 19b | 19c | 20 |
|---|---|---|---|---|
| | [wt-%] | [wt-%] | [wt-%] | [wt-%] |
| **Phase A** | | | | |
| Marlinat 242/70 | 2.8 | 5.2 | 3.9 | |
| Myrj 59P Pharma | 1.3 | 2.3 | 1.7 | |
| Lactic Acid (90%) | 0.0 | 0.1 | 0.1 | |
| Glycerine | 3.9 | 7.2 | 5.3 | |
| Demin. water | 1.2 | 2.2 | 1.6 | |
| Clucopon SC 215 | | | | 58.6 |
| **Phase B** | | | | |
| Cutina CP | 23.2 | 42.7 | 50.1 | |
| Parsol TX | 9.3 | 17.1 | 20.0 | |
| Wacker-Belsil DM 1 plus | | | | 33.1 |
| UV-TITAN M160 | | | | 8.3 |
| **Phase C** | | | | |
| Demin. water | 57.7 | 23.1 | 17.1 | |
| Keltrol | 0.2 | 0.1 | 0.1 | |
| Dekaben C | 0.4 | 0.2 | 0.1 | |
| **Sum:** | 100.0 | 100.0 | 100.0 | 100.0 |

### Reference example 21

An OW-lotion is prepared, by preparing separately the individual phases using conventional vessels and stirrers. Phases A and B are heated to 80°C. Phase A is added to phase B. The mixture is homogenised for 1 min. Phase C is heated to 80°C and added to the mixture of A+B and homogenised. The mixture is let to cool under critical gel network temperature and homogenized further for 1 min. Subsequently the mixture is cooled to 25°C and homogenized. Mixture is neutralized with 10 w-% NaOH to pH about 6.5. Phase D is added and the mixture is further homogenised. At the end the phase E is added by stirring with a spatula. The thus obtained emulsion, a basic OW-lotion is subsequently cooled or left to cool to room temperature.

Components employed and the phases are shown in table 4.

**Table 4**

| Example | | | | 21 |
|---|---|---|---|---|
| **Trade name** | | | | [wt.-%] |
| **Phase A** | | | | |
| Kemira | Glycerine stearate, cetearyl alcohol, sodium stearoyl lactylate | | NPT | 5.00 |
| Cetiol SN | Cetearyl isononanoate | | Cognis | 8.00 |
| Arlamol HD | Heptamethylnonane | | Uniqema | 5.00 |
| Tocopherolacetat | Vitamin E acetate | | Roche | 1.00 |
| Beeswax | | | | |
| **Phase B** | | | | |
| demin. Water | | | | 15.00 |
| Keltrol | Xanthan gum | | NutraSweet | 0.20 |
| Ultrez21 | | | | 0.30 |

(continued)

| Phase C | | | | |
|---|---|---|---|---|
| Pricerine 9091 | Glycerine | | Uniqema | 5.00 |
| demin. Water | | | | 58.40 |
| **Phase C1** | | | | |
| 10% NaOH | | | | q.s. |
| **Phase D** | | | | |
| Allianz OPT | | | | 2 |
| **Phase E** | | | | |
| Phenonip | Phenoxyethanol (and) Methylparaben (and) | | Nipa | 0.10 |
| | Ethylparaben (and) Butylparaben (and) | | | |
| | Propylparaben (and) Isobutylparaben | | | |
| **Sum** | | | | **100.00** |

**Example 22**

This example demonstrates the stability of OW-lotion and sun lotion formulas wherein liquid-liquid $TiO_2$ dispersions according to invention are incorporated. The assessment of the physical stability of the sun lotion is made visually and by measuring the particle size distribution. The visual assessment of stability is made using the following grading:

**Observation notation:**

| | |
|---|---|
| 1 | = OK |
| 2 | = inhomogeneous |
| 3/W | = visible water separation |
| 3/O | = visible oil separation |
| 4/W | = measurable water separation |
| 4/O | = measurable oil separation |
| 5 | = total separation |

The results obtained are shown in table 5.

## Table 5

| Example | 22a | 22b | 22c | 22d | 22e |
|---|---|---|---|---|---|
| | Gel4 | 50% Gel4 in OW | 35% Gel4 in OW | 20% Gel4 in OW | Basic OW |
| | 28% TiO2 | 14% TiO2 | 9.8% TiO2 | 5.6% TiO2 | |
| **Basic OW-lotion** | 0 | 50 | 65 | 80 | 100 |
| | | | | | |
| **TiO2 Nanogel** | 100 | 50 | 35 | 20 | 0 |
| **28% UV-TITAN M160** | | | | | |
| **In-Vitro SPF** | | 24.2 ± 3.6 | 15.6 ± 1.7 | 8.7 ± 0.9 | |
| **ratio** | | 1.73 | 1.59 | 1.55 | |
| | | | | | |
| **PSA (Volume)** | | | | | |
| Median ($D_{50}$) without Sonic [µm] | 0.34 | 0.82 | 1.5 | 2.1 | 2.7 |
| Diameter < 1µm [%] | 99.8 | 56.6 | 27.8 | 1.62 | 0 |
| Specific surface area [$cm^2/cm^3$] | 2.01E+05 | 1.19E+05 | 6.90E+04 | 3.00E+04 | 2.23E+04 |
| Deviation [µm] | 0.15 | 1.1 | 1.1 | 1.0 | 2.4 |
| **PSA (Volume)- after 11d** | | | | | |
| Median ($D_{50}$) without Sonic [µm] | | 1.1 | 1.7 | 2.1 | |
| Diameter < 1µm [%] | | 44.9 | 13.0 | 0.73 | |
| Specific surface area [$cm^2/cm^3$] | | 1.04E+05 | 4.80E+04 | 2.98E+04 | |
| Deviation [µm] | | 0.96 | 1.0 | 0.89 | |
| Median ($D_{50}$) with Sonic [µm] | | 0.80 | 1.3 | 1.7 | |
| Diameter < 1µm [%] | | 58.8 | 37.2 | 6.4 | |
| Specific surface area [$cm^2/cm^3$] | | 1.26E+05 | 8.37E+04 | 3.68E+04 | |
| Deviation [µm] | | 0.82 | 0.96 | 0.77 | |
| **Rheology** | | | | | |
| Yield point [Pa] | 32.4 | 1.40 | 3.37 | 4.34 | 20.9 |
| Viscosity [mPas] | 78 400 | 5400 | 8600 | 11700 | 42400 |

## Table 5, cont.

| Stability test | | | | | |
|---|---|---|---|---|---|
| **RT** | | | | | |
| 3 Days | 1 | 1 | 1 | 1 | 1 |
| 1 Week | 1 | 1 | 1 | 1 | 1 |
| 2 Weeks | 1 | 1 | 1 | 1 | 1 |
| 3 Weeks | 1 | 1 | 1 | 1 | 1 |
| 1 Month | 1 | 1 | 1 | 1 | 1 |
| 2 Month | 1 | 1 | 1 | 1 | 1 |
| 3 Month | 1 | 1 | 1 | 1 | 1 |
| **40°C** | | | | | |
| 3 Days | 1 | 1 | 1 | 1 | 1 |
| 1 Week | 1 | 1 | 1 | 1 | 1 |
| 2 Weeks | 1 | 1 | 1 | 1 | 1 |
| 3 Weeks | 1 | 1 | 1 | 1 | 1 |
| 1 Month | 1 | 1 | 1 | 1 | 1 |
| 2 Months | 1 | 1 | 1 | 1 | 1 |
| 3 Months | 1 | 1 | 1 | 1 | 1 |
| **50°C** | | | | | |
| 3 Days | 1 | 1 | 1 | 1 | 1 |
| 1 Week | 1 | 1 | 1 | 1 | 1 |
| 2 Weeks | 1 | 1 | 1 | 1 | 1 |
| 3 Weeks | 1 | 1 | 1 | 1 | 1 |
| 1 Month | 1 | 1 | 1 | 1 | 1 |
| 2 Months | 1 | 1 | 1 | 1 | 1 |
| 3 Months | 1 | 3/O | 1 | 1 | 3/O |
| **-18°C/40°C** | | | | | |
| 1 x | 1 | 1 | 1 | 1 | 1 |
| 2 x | 1 | 1 | 1 | 1 | 1 |
| 3 x | 1 | 1 | 1 | 1 | 1 |
| 4 x | 1 | 1 | 1 | 1 | 1 |
| 5 x | 1 | 1 | 1 | 1 | 1 |

**Example 23**

This example demonstrates the stability of OW-lotion and sun lotion formulas wherein solid-liquid $TiO_2$ dispersions according to invention are incorporated. The assessment of the physical stability of the sun lotion is made visually and by measuring the particle size distribution similarly to example 22.

The obtained results are shown in table 6.

<div align="center">Table 6</div>

| Example | 23a | 23b | 23c |
|---|---|---|---|
| | **9% TiO2** | **5% TiO2** | **Basic OW** |
| **Basic OW-lotion** | 0 | 55 | 100 |
| | | | |
| **Kemspheres with TiO2** | 100 | 45 | 0 |
| 9% UV-TITAN X290 | | | |
| **In-Vitro SPF - vitro skin** | **18.3 $\pm$ 2.3** | **14.4 $\pm$ 0.8** | |
| UVA / UVB Ratio | | 0.63 | |

(continued)

| Example | 23a | 23b | 23c |
|---|---|---|---|
| | **9% TiO2** | **5% TiO2** | **Basic OW** |
| Factor | 2.03 | 2.88 | |
| **PSA (Volume)** | | | |
| Median ($D_{50}$) without Sonic [$\mu$m] | 0.41 | 0.63 | 4.7 |
| Deviation [$\mu$m] | 0.17 | 0.41 | 3.6 |
| Diameter < 1 $\mu$m [%] | 99.2 | 86.7 | 1.5 |
| Specific surface area [$cm^2/cm^3$] | 1.73E+05 | 1.33E+05 | 2.10E+04 |
| Median ($D_{50}$) with Sonic [$\mu$m] | 0.39 | 0.52 | 1.5 |
| Deviation [$\mu$m] | 0.15 | 0.29 | 0.70 |
| Diameter < 1 $\mu$m [%] | 99.6 | 93.9 | 24.2 |
| Specific surface area [$cm^2/cm^3$] | 1.78E+05 | 1.48E+05 | 4.90E+04 |
| **Rheology** | | | |
| Yield point [mPa] | 130 | 1100 | 18600 |
| Viscosity [mPas] | 6000 | 7000 | 32400 |
| **Stability test** | | | |
| **RT** | | | |
| 3 Days | | 1 | |
| 1 Week | | 1 | |
| 2 Weeks | | 1 | |
| 3 Weeks | | 1 | |
| 1 Month | | 1 | |
| 2 Month | | 1 | |
| 3 Month | | 1 | |
| **40°C** | | | |
| 3 Days | | 1 | |
| 1 Week | | 1 | |
| 2 Weeks | | 1 | |
| 3 Weeks | | 1 | |
| 1 Month | | 1 | |
| 2 Months | | 1 | |
| 3 Months | | 1 | |
| **50°C** | | | |
| 3 Days | | 1 | |
| 1 Week | | 1 | |
| 2 Weeks | | 1 | |
| 3 Weeks | | 1 | |

(continued)

| Stability test | | | |
|---|---|---|---|
| RT | | | |
| 1 Month | | 1 | |
| 2 Months | | 1 | |
| 3 Months | | 1 | |
| -18°C/40°C | | | |
| 1 x | | 1 | |
| 2 x | | 1 | |
| 3 x | | 1 | |
| 4 x | | 1 | |
| 5 x | | 1 | |

**Example 24**

A sun lotion formula with liquid-liquid $TiO_2$ dispersion is prepared by first preraring the individual phases separately using conventional vessels and stirrers. Phase B is prepared without Antaron V220. Phases A and B are then heated to 80°C. Phase A is added to phase B. The mixture is homogenised for 1 min. Phase C is heated to 80°C and added to the mixture of A+B and homogenised. The mixture is let to cool under critical gel network temperature and homogenized further for 1 min. Subsequently the mixture is cooled to 25°C and homogenized. Mixture is neutralized with 10 w-% NaOH to pH about 6.5. Phase D is added and the mixture is further homogenised. At the end the phase E is added by stirring with a spatula.

Components employed and the phases are shown in table 7 and the results obtained are in table 8.

**Table 7**

| Phase A | |
|---|---|
| Pationic SCL | 0.20 |
| Lanette O | 0.60 |
| Tegin M (TD-4034) | 0.75 |
| Stearin acid | 0.75 |
| Lipacide PVB | 0.25 |
| Witconol TN | 2.60 |
| Miglyol 812N | 1.00 |
| Crodamol OP | 0.55 |
| DC-Fluid 345 | 0.60 |
| Candlenut oil | 2.00 |
| Vitamin E Acetate | 1.00 |
| Neo Helipan E1000 (B) | 7.60 |
| Parsol 1789 | 1.00 |
| Phase B | |
| Witconol TN | 2.70 |
| Miglyol 812N | 1.00 |

(continued)

| Phase B | |
|---|---|
| Crodamol OP | 0.70 |
| DC-Fluid 345 | 0.70 |
| Antaron V-220 | 1.00 |
| **Phase C** | |
| demin Water | **41.54** |
| EDTA Di-Natrium | 0.20 |
| Ethanol | 8.00 |
| Glycerine | 8.00 |
| Ultrez 10 | 0.15 |
| **Phase D** | |
| Keltrol | 0.25 |
| **Phase E** | |
| Ti02-nanogel | **16.86** |
| (3.4% TiO2 in formula) | |
| **Phase F** | |
| Ethanol | |
| Glycerine | |
| **Sum:** | 100.00 |

**Table 8**

| PSA (Volume) after production | | PSA (Volume) after 5 x TGC | |
|---|---|---|---|
| Median ($D_{50}$) without Sonic [μm] | 0.99 | Median ($D_{50}$) without Sonic [μm] | 0.93 |
| Std [μm] | 0.43 | Diameter < 1μm [%] | 57.4 |
| Diameter < 1μm [%] | 50.6 | Specific surface area [$cm^2/cm^3$] | 6.82E+04 |
| Specific surface area [$cm^2/cm^3$] | 6.40E+04 | Deviation [μm] | 0.45 |
| Median ($D_{50}$) with Sonic [μm] | 0.77 | Median ($D_{50}$) with Sonic [μm] | 0.69 |
| Std [μm] | 0.37 | Diameter < 1μm [%] | 83.3 |
| Diameter < 1μm [%] | 74.6 | Specific surface area [$cm^2/cm^3$] | 9.36E+04 |
| Specific surface area [$cm^2/cm^3$] | 8.74E+04 | Deviation [μm] | 0.32 |
| **Rheology** | | | |
| Yield point [mPa] | 5400 | | |
| Viscosity [mPas] | 25900 | | |
| **PSA (Volume) RT** | after 1 Month | **PSA (Volume) 40°C** | |
| Median ($D_{50}$) without Sonic [μm] | 0.84 | Median ($D_{50}$) without Sonic [μm] | 0.99 |
| Diameter < 1μm [%] | 70.4 | Diameter < 1μm [%] | 50.8 |
| Specific surface area [$cm^2/cm^3$] | 7.42E+04 | Specific surface area [$cm^2/cm^3$] | 6.35E+04 |
| Deviation [μm] | 0.36 | Deviation [μm] | 0.62 |
| Median ($D_{50}$) with Sonic [μm] | 0.63 | Median ($D_{50}$) with Sonic [μm] | 0.68 |
| Diameter < 1μm [%] | 93.0 | Diameter < 1μm [%] | 86.9 |
| Specific surface area [$cm^2/cm^3$] | 9.99E+04 | Specific surface area [$cm^2/cm^3$] | 9.43E+04 |
| Deviation [μm] | 0.22 | Deviation [μm] | 0.27 |
| **PSA (Volume) RT** | after 2 Months | **PSA (Volume) 40°C** | |
| Median ($D_{50}$) without Sonic [μm] | 0.68 | Median ($D_{50}$) without Sonic [μm] | 1.0 |
| Diameter < 1μm [%] | 91.0 | Diameter < 1μm [%] | 48.1 |
| Specific surface area [$cm^2/cm^3$] | 9.27E+04 | Specific surface area [$cm^2/cm^3$] | 6.16E+04 |
| Deviation [μm] | 0.22 | Deviation [μm] | 0.42 |
| Median ($D_{50}$) with Sonic [μm] | 0.56 | Median ($D_{50}$) with Sonic [μm] | 0.69 |
| Diameter < 1μm [%] | 98.8 | Diameter < 1μm [%] | 87.9 |
| Specific surface area [$cm^2/cm^3$] | 1.11E+05 | Specific surface area [$cm^2/cm^3$] | 9.19E+04 |
| Deviation [μm] | 0.15 | Deviation [μm] | 0.25 |

## Table 8, cont.

| PSA (Volume) RT | after 3 Months | PSA (Volume) 40°C | |
|---|---|---|---|
| Median ($D_{50}$) without Sonic [µm] | 0.88 | Median ($D_{50}$) without Sonic [µm] | 1.5 |
| Diameter < 1µm [%] | 63.5 | Diameter < 1µm [%] | 17.0 |
| Specific surface area [$cm^2/cm^3$] | 7.06E+04 | Specific surface area [$cm^2/cm^3$] | 4.31E+04 |
| Deviation [µm] | 0.64 | Deviation [µm] | 0.99 |
| Median ($D_{50}$) with Sonic [µm] | 0.77 | Median ($D_{50}$) with Sonic [µm] | 1.3 |
| Diameter < 1µm [%] | 78.9 | Diameter < 1µm [%] | 29.8 |
| Specific surface area [$cm^2/cm^3$] | 8.31E+04 | Specific surface area [$cm^2/cm^3$] | 5.09E+04 |
| Deviation [µm] | 0.30 | Deviation [µm] | 0.87 |
| **Stability test, visual assessment** | | | |
| **RT** | | **40°C** | |
| 1/3 Days | 1 | 1/3 Days | 1 |
| 1 Week | 1 | 1 Week | 1 |
| 2 Weeks | 1 | 2 Weeks | 1 |
| 3 Weeks | 1 | 3 Weeks | 1 |
| 1 Month | 1 | 1 Month | 1 |
| 2 Months | 1 | 2 Months | 1 |
| 3 Months | 1 | 3 Months | 1 |
| **-18°C/40°C** | | | |
| 1 x | 1 | | |
| 2 x | 1 | | |
| 3 x | 1 | | |
| 4 x | 1 | | |
| 5 x | | | |

**Claims**

1. Membrane-structured solid nanoparticles (MSSNs) and have a combination of active compound vehicles and emulsifiers as such to form membranes which infiltrate the entire nanoparticles so that there are emulsifiers in the interior and on the surface of said nanoparticles **characterised in that** said active compound comprises an ultrafine titanium dioxide active compound.

2. The MSSNs according to claim 1 **characterised in that** the ultrafine titanium dioxide active compounds are uniformely devided inside said MSSN structures and exhibit a monomodular particle size distribution when measured from a dispersion thereof.

3. The MSSNs according to claim 1 or claim 2 **characterised in that** ultrafine titanium dioxide active compounds of the MSSN structure have an average particle diameter $D_{50}$ in the range from 100 nm to 800 nm, and preferably the width of the distribution is 0.15 µm or less.

4. The MSSNs according to any of the claims 1-3 **characterised in that** the crystal size of titanium dioxide in said ultrafine titanium dioxide active compound is less than 100 nm, preferably from 3 to 80 nm, more preferably from 8 to 35 nm.

5. The MSSNs according to any of the claims 1-4 **characterised in that** it comprises in addition to ultrafine titanium dioxide active compound zinc oxide and/or organic absorber.

6. The MSSNs according to any of the claims 1-5 **characterised in that** said ultrafine titanium dioxide active compound comprises a surface treatment with at least one of the following compounds, aluminium compound, zirconium coumpound, silicon containing inorganic compound, sulphuric acid or organic additive.

7. An aqueous dispersion comprising the MSSNs of any of the claims 1-6.

8. The dispersion according to claim 7 **characterized in that** said ultrafine titanium dioxide active compound is embedded in liquid oily vehicle.

9. The dispersion of claim 8 **characterized in that** the amount of ultrafine $TiO_2$ active compound based on the total amount of aqueous $TiO_2$ vehicle dispersion, is from 0.1% to 50%, preferably from 0.1% to 40% by weight, more preferably from 1 % to 40% by weight, most preferably from 1 % to 35% such as from 1 % to 30% by weight.

10. The dispersion according to claim 7 **characterized in that** ultrafine titanium dioxide active compound is embedded in solid waxy vehicle.

11. The dispersion according to claim 10 **characterized in that** the amount of ultrafine $TiO_2$ active compound based on the total amount of aqueous $TiO_2$ vehicle dispersion is from 0.1% to 50% by weight, preferably from 0.1% to 30% by weight, more preferably from 1% to 20%, most preferably from 1% to 10% by weight.

12. A method for producing a dispersion comprising membrane-structured solid nanoparticles (MSSNs) having a combination of ultrafine titanium dioxide active compound vehicle particles and emulsifiers as such to form membranes which infiltrate the entire nanoparticles so that there are emulsifiers in the interior and on the surface of said nanoparticles and which comprise ultrafine titanium dioxide active compounds wherein said method comprises the steps of

   a. mixing said ultrafine titanium dioxide active compound with the wax-, polymer- or lipid-based ultrafine titanium dioxide active compound vehicle and at least one emulsifier at a temperature above the melting point or softening point of said ultrafine titanium dioxide active compound vehicle, in order to form phase B, and
   b. mechanically mixing said phase B with an aqueous phase A, which optionally comprises an emulsifier, at a temperature above the melting point or softening point of said ultrafine titanium oxide active compound vehicle, without high-pressure homogenization, leading to formation of a lyotropic liquid-crystalline mixed phase A+B, and
   c. diluting said mixed phase A+B with an aqueous phase C, which optionally comprises an emulsifier using stirring but not high-pressure homogenization, to achieve a desired final concentration of said dispersion.

13. The method of claim 12 **characterised in that** the mechanical mixing in step b and the stirring in step c take place with agitators which have a peripheral speed in the range from 1 to 20 m/s and produce a lamellar flow.

14. The method of claim 12 or claim 13 **characterised in that** the weight ratio of phase B to phase A in step b is from 1:5 to 5:1.

15. The method of any of the claims 12-14 **characterised in that** the average diameter of said titanium dioxide active compound vehicle particles are from 50 to 1000 nm.

16. Use of the MSSNs or MSSN dispersions of any of the claims 1-11 or the aqueous dispersion of any of the claims 7-10 for OM/ or W/O dispersions, preferably in cosmetics and coatings.

**Figure 1**. UV/VIS absorption behaviour of the ultrafine TiO$_2$ active compound used in the present invention.

a.

b.

**Figure 2a**. A schematic graph of the particle size distribution of a conventional dispersion containing agglomerated ultrafine $TiO_2$.TiO2-MSSNs in a dispersion according to the invention.

**Figure 2b**. A schematic graph of the particle size distribution of a dispersion containing ultrafine $TiO_2$ in a dispersion of $TiO_2$-MSSNs according to the invention.

**Figure 3**. A schematic figure of the structure of a TiO₂-MSSN according to the invention.

**Figure 4**. The particle size distribution of a dispersion containing ultrafine $TiO_2$ in a dispersion of $TiO_2$-MSSNs for example 18i–j.

**Figure 5**. Impact of the particle size of TiO$_2$-MSSN on film formation.

**European Patent Office**

**EUROPEAN SEARCH REPORT**

Application Number

EP 08 15 6453

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | US 2006/257334 A1 (DAHMS GERD [DE] ET AL) 16 November 2006 (2006-11-16) * paragraphs [0010] - [0071]; claims 16,17,21,25,26 * | 1-8,10, 12-16 | INV. C01G23/047 A61K8/18 |
| A | G.H.DAHMS: "Membrane structured solid nanoparticles- a novel nanotechnology for delivery of cosmetic active ingredient" INTERNATIONAL JOURNAL OF COSMETIC SCIENCE, vol. 28, 2006, pages 70-71, XP002496137 * pages 70-71 * | 1-11 | |
| A | US 2006/024248 A1 (SPENGLER ERIC G [US] ET AL) 2 February 2006 (2006-02-02) * paragraphs [0029] - [0052] * | 1-16 | |

TECHNICAL FIELDS SEARCHED (IPC)

C01G
A61K

The present search report has been drawn up for all claims

| Place of search | Date of completion of the search | Examiner |
|---|---|---|
| The Hague | 17 September 2008 | Corrias, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 15 6453

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2008

| Patent document cited in search report | | Publication date | Patent family member(s) | | Publication date |
|---|---|---|---|---|---|
| US 2006257334 | A1 | 16-11-2006 | AU<br>CA<br>DE<br>EP<br>WO<br>JP<br>KR | 2004222631 A1<br>2519697 A1<br>10312763 A1<br>1605923 A2<br>2004082666 A2<br>2006520750 T<br>20050114255 A | 30-09-2004<br>30-09-2004<br>30-09-2004<br>21-12-2005<br>30-09-2004<br>14-09-2006<br>05-12-2005 |
| US 2006024248 | A1 | 02-02-2006 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 444798 A
- US 4880634 A

- WO 2004082666 A
- WO 2007036240 A


**Non-patent literature cited in the description**

- **H. Aro ; G. Dahms.** Compatibility of micro-fine titanium dioxide with organic UV filters. Cosmetic and Toiletries Manufacture Worldwide, 2004, 115-118

- *J. Microencapsulation,* 1999, vol. 16 (6), 751-767
- **J.R. Villalobos ; C.C. Mueller-Goymann.** *Proc. Int. Meeting on Pharm. Biopharm., and Pharm. Tech.,* 15 March 2004